(19) Europäisches Patentamt
European Patent Office
Office européen des brevets

(11) EP 2 098 228 A1

(12) EUROPEAN PATENT APPLICATION
published in accordance with Art. 153(4) EPC

(43) Date of publication:
**09.09.2009 Bulletin 2009/37**

(21) Application number: **07829811.4**

(22) Date of filing: **15.10.2007**

(51) Int Cl.:
***A61K 31/136*** (2006.01) ***A61K 8/41*** (2006.01)
***A61K 8/49*** (2006.01) ***A61K 31/5415*** (2006.01)
***A61P 29/00*** (2006.01) ***A61P 43/00*** (2006.01)
***A61Q 19/02*** (2006.01)

(86) International application number:
**PCT/JP2007/070077**

(87) International publication number:
**WO 2008/047758 (24.04.2008 Gazette 2008/17)**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **17.10.2006 JP 2006283050**

(71) Applicant: **Kabushiki Kaisha Hayashibara Seibutsu Kagaku Kenkyujo**
**Okayama-shi**
**Okayama 700-0907 (JP)**

(72) Inventors:
- **MIYAKE, Masaki**
  **Okayama-shi**
  **Okayama 7000907 (JP)**
- **KOHNO, Keizo**
  **Okayama-shi**
  **Okayama 7000907 (JP)**
- **SANO, Osamu**
  **Okayama-shi**
  **Okayama 7000907 (JP)**

(74) Representative: **Daniels, Jeffrey Nicholas**
**Page White & Farrer**
**Bedford House**
**John Street**
**London WC1N 2BF (GB)**

# (54) ANTIINFLAMMATORY AGENT COMPRISING 2-AMINOPHENOL OR DERIVATIVE THEREOF AS ACTIVE INGREDIENT

(57) An object of the present invention is to provide anti-inflammatory agent having an excellent effect and less adverse side-effects. The object is attained by providing an anti-inflammatory agent comprising 2-aminophenol or a derivative thereof as an effective ingredient.

EP 2 098 228 A1

**Description**

## TECHNICAL FIELD

The present invention relates to an anti-inflammatory agent, particularly, to an anti-inflammatory agent comprising 2-aminophenol or a derivative thereof as an effective ingredient.

## BACKGROUND ART

While inflammatory response is one of important biological defenses to protect living bodies from pathogens, excess inflammatory response is rather harmful because it also damages living tissues. Particularly, inflammatory responses without pathogens, such as autoimmune or allergic diseases, are highly harmful. Accordingly, various kinds of anti-inflammatory agents have been developed for inhibiting inflammatory responses. For example, nonsteroidal anti-inflammatory agents such as aspirin, diclofenac, indomethacin and mefenamic acid; and steroidal anti-inflammatory agents such as prednisolone, hydrocortisone acetate and difluprednate already have been used as medicines.

Major symptoms of inflammatory responses are "pain" and "swelling". "Pain" with inflammatory response is induced by prostaglandin. Since cyclooxygenase (herein af ter abbreviated as "COX") is required to produce prostaglandin, its inhibitors (COX inhibitors) are useful as an analgesic anti-inflammatory agent. COX, an enzyme for synthesizing prostaglandin from arachidonic acid, falls into two isoforms. Among which, an isozyme, COX-1, which is constitutively expressed in gastrointestinal tract, kidney and platelet, is essential for maintaining normal physiology. The other isozyme, COX-2, which is temporarily induced by inflammatory cytokines such as interleukin-1α and tumor necrosis factor α (TNF-α) and overexpressed by inflammation, is reported to be involved in inflammatory diseases such as rheumatism and arthritis, cancer, gastric ulcer, Alzheimer's disease and ovulation and delivery. COX inhibitors used as anti-inflammatory agents are intended to inhibit the COX-2 activity, however, many anti-inflammatory agents inhibit also the COX-1 activity resulting in adverse side-effects such as stomach ache. Accordingly, COX-2 selective inhibitors were developed in Europe and the United States, and had promise as anti-inflammatory agents with low adverse side-effects such as gastrointestinal and kidney damages. However, COX-2 selective inhibitors have safety concerns because patients with colonic adenomatous polyp administered with rofecoxib, one of COX-2 selective inhibitors, have high risks for cardiovascular diseases such as myocardial infarct as reported in Shinmura et al., "Possible mechanisms of cyclooxygenase (COX)-2 hazard: Is COX-2 in the cardiovascular system a friend or a foe?", Inflammation and Regeneration, Vol. 25, No. 6, 517-524 (2005). Consequently, they have not yet been approved as medicines in Japan.

"Swelling" with inflammation occurs by dilation of vessel in affected area and topical concentration of immunocompetent cells such as leukocyte, lymphocyte and macrophage. Since the dilation of vessel is induced by nitric oxide (NO), inhibitors of NO production are useful for alleviation of swelling. NO is produced by oxidation of L-arginine by nitric oxide synthase (NOS). NOS falls into two types, noninductive and inductive one. Among which, the inductive NOS (iNOS), which mainly exists in macrophage, endothelial cells and smooth muscle cells, is an important factor for inflammatory response. Consequently, inhibitors of activity or production of iNOS are effective in inhibiting NO production. $N^G$-nitro-L-arginine-methyl-ester, derivatives of isothiourea, 2-iminopiperidine and L-canavalin are quoted as inhibitors of iNOS activity.

As reported that COX-2 and iNOS are concurrently overexpressed in many inflammatory diseases, activities of these two enzymes thought to be highly concerning in inflammatory response. Consequently, simultaneous inhibition of COX-2 activity and iNOS activity is necessary for effective alleviation of "pain" and "swelling". However, conventional COX and NOS inhibitors have high specificity as inhibit either COX activity or iNOS activity. So anti-inflammatory agent that can concurrently alleviate "pain" and "swelling" is expected.

2-Aminophenol (aka "questiomycin B"), a compound represented by Chemical formula 1 described below, is easily converted into 2-aminophenoxazine-3-one (aka "questiomycinA") represented by Chemical formula 2 described below by oxidative polymerization. 2-Aminophenol and its derivatives are known beforehand as an antibacterial substance, particularly, 2-aminophenoxazine-3-one is known as the basic structure of actinomycin D, a strong anticancer agent. Motohashi et al., "Potential antitumor phenoxazines", Medicinal Research Reviews, Vol. 11, 239-294 (1991) disclosed that 2-aminophenoxazine-3-one and its derivatives have antitumor activities, and particularly Shimamoto et al., "Antitumor effects of a novel phenoxazine derivative on human leukemia cell lines in vitro and in vivo", Clinical Cancer Research, Vol. 7, 704-708 (2001) disclosed that one of its derivatives, 2-amino-4,4α-dihydro-4α,7-dimethyl-phenoxazine-3-one, has cytopathic activity on various kind of tumor cells. In more recent years, 2-aminophenoxazine-3-one is disclosed to be effective in the treatment for virus disease as described in Japanese Patent Kokai No. 2143101/2004, chlamydiosis as described in Japanese Patent Kokai No. 272334/2005, and gastrointestinal disease involved with genus *Helicobacter* as described in Japanese Patent Kokai No. 60325/2005. However, it is not known that 2-aminophenol derivatives have anti-inflammatory effect.

Chemical formula 1:

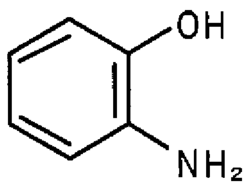

Chemical formula 2:

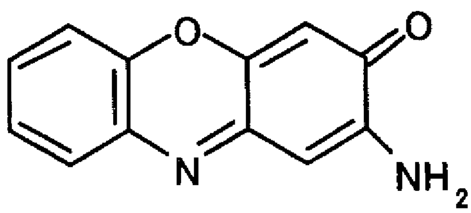

## DISCLOSURE OF INVENTION

An object of the present invention is to provide an anti-inflammatory agent with less adverse side-effects and higher improvement effect on inflammatory symptoms such as "pain" and "swelling" than hitherto known anti-inflammatory agents.

The inventors of the present invention dedicated to carry on the research and found that 2-aminophenol or its derivatives have inhibiting effect on prostaglandin E2 synthesis by inhibiting cyclooxygenase (COX) activity, inhibiting effect on nitric oxide synthesis in macrophage by inhibiting iNOS production, and inhibiting effect on degranulation of mast cell. And also it was found that 2-aminophenol or its derivatives have inhibiting effect on melanin synthesis in melanocyte and enhancing effect on collagen production, and the present invention has been accomplished.

The present invention attains the above object by providing an anti-inflammatory agent comprising 2-aminophenol or a derivative thereof.

In accordance with the present invention, anti-inflammatory agents with lower adverse side-effects and higher alleviating effect on inflammatory symptoms than hitherto known anti-inflammatory agents are provided. And they are useful as skin-whitening cosmetics in the form of an external dermatological agent.

## BEST MODE FOR CARRYING OUT THE INVENTION

2-Aminophenol as referred to as in the present invention is a compound represented by Chemical formula 1 described below and its commercially available products can be used without regard to their sources. Derivatives of 2-aminophenol as referred to as in the present invention means 2-aminophenoxazine-3-one, which is an oxidative polymer of 2-aminophenol, and its derivatives having the basic structure thereof and have the same or higher effect as of 2-aminophenol. 2-Aminophenoxazine-3-one can be produced by proper methods for extraction and purification from plants or bacteria rich in it or synthesized by oxidative polymerization of its precursor, 2-aminophenol. For example, it can be synthesized by the method of allowing 2-aminophenol to react with trivalent iron ions such as potassium ferricyanide as described in Japanese Patent Kokai No. 2878/2003 or the method of allowing 2-aminophenol to react with human or bovine hemoglobin.

Chemical formula 1:

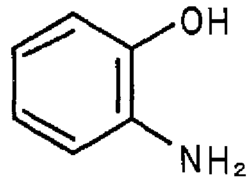

Chemical formula 2:

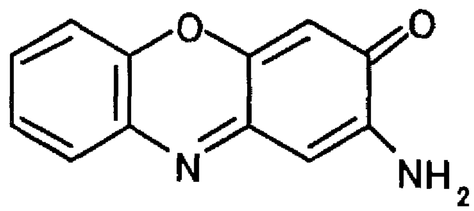

As derivatives of 2-aminophenoxazine-3-one, naturally occurring such derivatives can be quoted as follows: 2-Amino-7-hydroxy-phenoxazine-3-one ($R_5$ is hydroxyl group and $R_1$ to $R_4$ are hydrogens in General formula 1 described below), 2-amino-7-methoxy-phenoxazine-3-one ($R_5$ is methoxy group and $R_1$ to $R_4$ are hydrogens in General formula 1 described below), 2-acetylamino-phenoxazine-3-one ($R_1$ is acetyl group and $R_2$ to $R_5$ are hydrogens in General formula 1 described below), 2-acetylamino-7-hydroxy-phenoxazine-3-one ($R_1$ is acetyl group, $R_5$ is hydroxyl group and $R_2$ to $R_4$ are hydrogens in General formula 1 described below), 2-(N-hydroxyl)acetylamino-phenoxazine-3-one ($R_1$ is acetyl group, $R_2$ is hydroxyl group and $R_3$ to $R_5$ are hydrogens in General formula 1 described below), 2- (2-hydroxyacetyl) amino-phenoxazine-3-one ($R_1$ is hydroxyacetyl group and $R_2$ to $R_5$ are hydrogens in General formula 1 described below), 2-acetylamino-7-methoxy-phenoxazine-3-one ($R_1$ is acetyl group, $R_5$ is methoxy group and $R_2$ to $R_4$ are hydrogens in General formula 1 described below), 7-hydroxy-2-(2-hydroxyacetyl) amino-phenoxazine-3-one ($R_1$ is hydroxyacetyl group, $R_5$ is hydroxyl group and $R_2$ to $R_4$ are hydrogens in General formula 1 described below), 2-amino-4,6,7-trimethoxy-phenoxazine-3-one ($R_3$ to $R_5$ are methoxy group and $R_1$ and $R_2$ are hydrogens in General formula 1 described below). They can be artificially transferred to saccharides to obtain glycosides or bound to water-soluble polymers such as polyethylene glycols and pullulan.

General formula 1

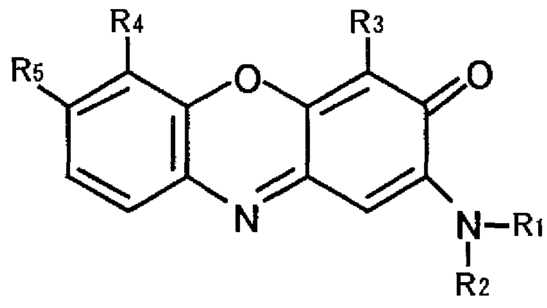

(Wherein $R_1$ and $R_2$ independently represent hydrogen (H), hydroxyl group (OH), acetyl group ($COCH_3$), and hydroxyacetyl group ($COCH_2OH$); and $R_3$ to $R_5$ independently represent hydrogen (H), hydroxyl group (OH) and methoxy group ($OCH_3$))

The inhibiting effect on nitric oxide synthesis as referred to as in the present invention is exerted by quantitatively decreasing iNOS in cells, and can be assayed by measuring the amount of NO produced by iNOS in the cells having activity of nitric oxide synthesis such as macrophage cells when the compounds of the present invention are added to the cells. As macrophage cells, RAW264.7 cell strain from mouse or macrophage cells obtained from laboratory animals such as mouse can be used. The amount of produced NO can be measured by conventional Griess method. In Griess method, the quantity of $NO_2^-$, metabolic product of NO, is determined by measuring absorbance at 540 nm of the red azo dye produced by diazotization coupling reaction when NO is added to Griess reagent, a mixture of sulfanilamide and N-(1-naphthyl)ethylenediamine.

The inhibiting effect on cyclooxygenase (COX) activity as referred to as in the present invention can be determined by measuring the amount of prostaglandin $E_2$ (PGE2) produced by adding the compound of the present invention as test samples in the presence of COX-1 or COX-2 and arachidonic acid compared with by adding control sample. Fifty percent inhibitory concentration ($IC_{50}$) means the concentration of test sample necessary for inhibiting PGE2 synthesis by 50% compared to non-inhibitory control sample. It can be also determined by measuring the amount of PGE2 produced by adding test samples to cells having cyclooxygenase activity. COX-1/COX-2 ratio means the ratio of $IC_{50}$ for COX-2 to $IC_{50}$ for COX-1, and the higher the ratio is, the more specific to COX-2 the agent is.

The inhibiting effect on melanin synthesis as referred to as in the present invention can be assayed as follows: Melanocytes such as mouse melanoma cell strain B16 are cultured with the compound of the present invention for proper period, and then the amount of melanin in the cells is determined by measuring the absorbance, for example, at 400 to 500 nm.

As described above, 2-aminophenol and its derivatives used in the present invention, which have inhibiting effect on NO synthesis, cyclooxygenase activity and degranulation of mast cell and basophillic cell, are feasible as anti-inflammatory agents, antiallergic agents and antiatopic agents for wide variety of uses as foods and drinks, cosmetics, quasi drugs and medicines. Since they also have promoting effect on differentiation of lymphatic T-cell into Th2-cell, they can be used as preventives and therapeutic agents for autoimmune diseases such as rheumatism and psoriasis. Since they also have inhibiting effect on melanin synthesis and enhancing effect on collagen production, they are feasible as skin-whitening and skin-beautifying cosmetics having antiaging effect when used in the form of an external dermatological agent. When the agent of the present invention is used in the form of an external dermatological agent, the content of 2-aminophenol or its derivatives as an effective ingredient in the anti-inflammatory agent is usually 0.00002 to 1% (w/w), preferably 0.0001 to 0.5% (w/w). The dose can be determined according to the condition of the skin, and a suitable dose is usually 0.1 $\mu$g to 10 mg, preferably, 1 $\mu$g to 5 mg per 1 $cm^2$ of the skin in once or several times a day.

The external dermatological agent described above can comprise substances having skin-whitening effect or enhancing effect on collagen production in additionto2-aminophenolanditsderivatives. L-Ascorbic acid and its salts; alkoxysalicylic acid and its salts; tranexamic acid and its salts; ellagic acid and its salts; linoleic acid and its salts; kojic acid and its salts; resorcinol, glutathione, cysteine, hydroquinone, tetrahydrocurcuminoid, or their derivatives can be quoted as the substances. Plant extracts from camomile or indigo, containing the above substances having skin-whitening effect, can be used. Among which, combinational use with L-ascorbic acid derivatives, kojic acid or trehalose is particularly preferable because they synergistically heighten skin-whitening effect and/or enhancing effect on collagen production.

The external dermatological agent of the present invention can comprise various kinds of any materials generally used for cosmetics, quasi drugs and medicines in addition to the above ingredients. For example, water, ethanol, glycerol, humectants, oily substances, emulsifiers, emulsion stabilizers, thickeners, antiseptics, fine particles, pigments, dyes, ultraviolet absorbers, ultraviolet scatterers, pH adjusters, flavors and medicinal substances canbe quoted.

The application of the external dermatological agent of the present invention is not restricted to general dermatological cosmetics, it is intended to all-round external dermatological agent as quasi drugs and medicines, for example, skin-whitening, reducing wrinkle and fleck, therapy for mole, nevus of ota, flat nevus, sunburn, burn injury, insect bite, bruise and atopic dermatitis. The agent can be used in any dosage form according to its purposes without restrictions. For example, it can be formed into liquid, powder, solid, emulsion, cream and gel and it is applicable to skin-care products such as lotion, essence, emulsion, cream, facial mask, massaging preparation, facial wash, cleansing preparation and sunburn preventive, body-care cosmetics such as body powder and body lotion, make-up cosmetics such as foundation, face powder and eye color and lipstick, ointment, aerosol and plaster.

When the present invention is applied to anti-inflammatory agent, an applicable form for oral administration can be feasible as well as the forms for an external dermatological agent described above, for example, those of a tablet, troche, pill, aqueous suspension, oily suspension, dispersant powder or granule, emulsion, hard capsule, soft capsule, syrup and elixir are quoted. The agent is applicable to local administration, parenteral administration, inhalation spray or rectal administration in a form suitable for these.

The anti-inflammatory agent of the present invention can be combinationally used with substances having anti-inflammatory effect described above and administered together with medicines having other effects. For example, one or more substances selected from analgesics such as acetaminophen and phenacetin, enhancers such as caffeine; decongestants such as phenylephrine, phenylpropanolamine, pseudoephedrin, oxymetazoline, epinephrine, naphazoline, xylometazoline, propylhexedrine and levodesoxyephedrin; antitussives such as codeine, hydrocodone, caramiphen, carbetapentane and dextromethorphan; H2-antagonist; aluminum hydroxide; magnesium hydroxide; simethicone; hydragogue; analgesic antihistamic agent; and nonanalgesic antihistamic agent can be used in combination.

The ant i - inf lammatory agent of the present invention is applicable to various kinds of inflammatory diseases. The inflammatory diseases means those with symptoms of inflammation, for example, rheumatoid arthritis, rheumatoid spondylitis, osteoarthritis, gouty arthritis, colitis, enteritis, Crohn's disease, Guillain-Barre syndrome, scleroderma, fibrosis, dermatitis, psoriasis, vascular edema, eczematous dermatitis, fast-proliferative dermatitis, glomerular nephritis, nephritis, gastritis, pancreatitis, conjunctivitis, rhinitis, gingivitis, gum disease, Alzheimer's disease, atherosclerosis, angiitis, phlebitis, arteritis, aortitis, post-PTCA restenosis, post-bypass surgery restenosis, various allergosis containing post-transplant rejection, anaphylaxis, blood poisoning, thrombosis, ischemia/reperfusion disorder and atopy can be quoted. Since the anti-inflammatory agent of the present invention inhibits PGE2 production, it has effect on diseases accompanied by osteolysis, for example, rheumatoid arthritis, gum disease and osteoporosis. The anti-inflammatory agent of the present invention can be used as an analgesic, antipyretic and preventive and therapeutic agent of nervous diseases containing manic-depressive psychoses as well as for therapy of inflammatory diseases. The anti-inflammatory agent of the present invention can be also used combinationally with known anticancer agents as therapeutic agents of various cancers accompanied by overexpression of COX-2.

The anti-inflammatory agent of the present invention can be administered in a dosage-unit form preparation containing pharmaceutically acceptable bases, additives and vehicles. It can be also applied to domestic animals, domestic fowls and pets such as mouse, rat, horse, bovine, sheep, hog, dog, cat and fowl as well as for preventing and treating human diseases.

The content of 2-aminophenol or its derivatives as an effective ingredient in the anti-inflammatory agent of the present invention can be arbitrarily determined according to symptom, formulation, dosage form and intended animal. It is usually 0.0001 to 10% (w/w), preferably, 0.0001 to 1% (w/w). The dose can be arbitrarily decided according to symptom, formulation and dosage form. The daily dose is generally 0.01 μg to 25 mg/kg, preferably 0.1 μg to 5 mg/kg administered once or several times a day.

The following experiments explain the present invention in detail.

Experiment 1

Synthesis of 2-aminophenoxazine-3-one

2-Aminophenoxazine-3-one was synthesized from 2-aminophenol. Five hundred and fifty-five mg (5 mmol) of 2-aminophenol (commercialized by Wako Pure Chemical Industries, Ltd., Osaka, Japan) was suspended in 50 ml of distilled water, admixed with 225 ml of 0.1 N hydrochloric acid, and then adjusted to pH 7.0 with 0.1 N sodium hydroxide solution. The solution was admixed with 500 ml of 5 mM potassium ferricyanide aqueous solution by drops for five minutes with stirring and allowed to react at 26 °C for 30 minutes. The reaction mixture was dried under reduced pressure to give a solid, and then the solid was dissolved in 450 ml of methanol. After centrifuged to remove insoluble matters, the solution was dried under reduced pressure again. The obtained solid was dissolved in 200 ml of ethylacetate, admixed with 200 ml of 0.005 N hydrochloric acid solution, stirred, and then an ethyl acetate layer was recovered with a separatory funnel. After the above procedure was repeated 3 times, the ethyl acetate layer was concentrated into 15 ml under reduced pressure. The resulting concentrated solution was purified with silica gel chromatography column ("Wakogel C-200" commercialized by Wako Pure Chemical Industries, Ltd., Osaka, Japan) and reversed phase C30 chromatography column ("Develosil C30" commercialized by Nomura Chemical Co., Ltd., Aichi, Japan or "HW-40F" commercialized by Tosoh Corporation, Kyoto, Japan) in conventional manner to obtain 220 mg of 2-aminophenoxazine-3-one.

Experiment 2

Inhibiting activity on NO and PGEs production by macrophage

RAW264.7 cells, a murine macrophage cell strain, were suspended in PRMI1640 medium supplied with 10% (v/v) fetal calf serum (hereinafter abbreviated as "FCS") to give a cell suspension with a cell concentration of $1 \times 10^6$ cells/ml. The suspension was inoculated to a 96-well microplate by 50 μl per well, admixed with 2-aminophenoxazine-3-one to give the final concentration of 1.6 to 100 μM, admixed with 2μg/ml of lipopolysaccharide and 10 IU/ml of IFN-γ as inducers, then filled up to 200 μl with the above medium. After cultured at 37 °C for 2 days, the number of the living cell was counted, and then the amount of NO in each culture supernatant was measured by conventional Griess method

and the amount of PGE2 was measured with "PGE2-EIA kit" (commercialized by Amersham Bioscience, Inc.) using an anti-PGE2 antibody. The comparative experiments were performed in the same way as described above using indomethacin (commercialized by Wako Pure Chemical Industries, Ltd., Osaka, Japan) and aspirin (commercialized by Wako Pure Chemical Industries, Ltd. , Osaka, Japan), which are COX inhibitors, and NS-398 (commercialized by Wako Pure Chemical Industries, Ltd., Osaka, Japan), which is a COX-2 selective inhibitor. The experimental system without the test samples described above was set as control. The results are in Table 1. In Table 1, "QA" means 2-aminophenoxazine-3-one (questiomycin A), "NS" means NS-398, "IN" means indomethacin, and "AS" means aspirin. NO production, PGE2 production and macrophage growth are indicated as relative levels versus controls.

Table 1

| Concentration (μM) | NO production (%) | | | | PGE2 production (%) | | | | Macrophage growth (%) | | | |
|---|---|---|---|---|---|---|---|---|---|---|---|---|
| | QA | NS | IN | AS | QA | NS | IN | AS | QA | NS | IN | AS |
| 0.16 | 85 | 90 | 86 | 101 | 76 | 4 | 4 | 98 | 117 | 95 | 102 | 103 |
| 0.31 | 78 | 89 | 84 | 102 | 45 | 4 | 4 | 99 | 121 | 97 | 104 | 100 |
| 0.63 | 70 | 87 | 81 | 102 | 13 | 2 | 4 | 79 | 127 | 99 | 108 | 95 |
| 1.3 | 54 | 85 | 82 | 93 | 6 | 1 | 3 | 47 | 127 | 102 | 117 | 85 |
| 2.5 | 32 | 85 | 80 | 95 | 1 | 1 | 1 | 33 | 119 | 100 | 120 | 86 |
| 5.0 | 19 | 88 | 80 | 90 | 3 | 5 | 2 | 8 | 112 | 105 | 122 | 89 |
| 10 | 14 | 84 | 78 | 86 | 2 | 2 | 1 | 3 | 86 | 110 | 120 | 89 |

As shown in Table 1, 2-aminophenoxazine-3-one ("QA" in Table 1) inhibited NO production and PGE2 production without lowering macrophage growth. On the other hand, the other anti-inflammatory agents in the comparative experiments did not exert considerable inhibiting effect on NO production whereas it exerted dominant inhibiting effect on PGE2 production. It suggested that these hitherto known anti-inflammatory agents are not expected to have improving effect on "swelling" even though they are expected to have improving effect on "pain".

In addition, the amount of iNOS in the cell extracts of this experiment was measured by western blotting analysis using an anti-iNOS antibody. The result showed that the amount of iNOS decreased depending on the 2-aminophenoxazine-3-one concentration. The inhibiting activity of 2-aminophenoxazine-3-one on NO production was estimated to be exerted by reducing the amount on iNOS.

Experiment 3

Inhibiting activity on COX-1 and COX-2

To verify the results of Experiment 2, it was investigated that whether the activityof COXs, essential enzymes for PGE2 synthesis system, can be inhibited. Using "COX Inhibitor Screening Assay Kit" (commercialized by Cayman Chemical Company), a reaction system containing COX-1 or COX-2 and arachidonic acid as substrate was admixed with 2-aminophenoxazine-3-one or 2-amnophenol as test samples to give the concentrations shown in Table 2 described below, and the amount of produced PGE2 was measured by EIA using an anti-PGE2 antibody to determine the COX activity. An experimental system without the test samples was set as control. The residual activity of COX-1 or COX-2 was determined as relative activity versus control. $IC_{50}$ of each test sample was calculated by plotting the residual activity at each concentration of the test samples in a graph. The results are in Table 2.

Table 2

| | 2-Aminophenoxazine-3-one | | | |
|---|---|---|---|---|
| | 1.9 μM | 3.8 μM | 7.6 μM | $IC_{50}$ |
| COX-1 | 89% | 59% | 44% | 6.44 μM |
| COX-2 | 95% | 37% | 29% | 4.83 μM |

(continued)

| | 2-Aminophenol | | | |
|---|---|---|---|---|
| | 8 $\mu$M | 16 $\mu$M | 32 $\mu$M | $IC_{50}$ |
| COX-1 | 66% | 73% | 42% | 28.0 $\mu$M |
| COX-2 | 72% | 60% | 47% | 27.7 $\mu$M |

As is evident from the results in Table 2, 2-aminophenol and 2-aminophenoxazine-3-one inhibited the activities of COX-1 and COX-2. The effect expressed in $IC_{50}$ of 2-aminophenoxazine-3-one was higher by 4 to 6-folds in a molar ratio, 2 to 3-folds in a mass ratio than by those of 2-aminophenol. The COX-1/COX-2 ratio was about 1 for 2-aminophenol and about 1.3 for 2-aminophenoxazine-3-one, the results indicated that their selectivity to COX-2 is higher than nonsteroidal COX inhibitors such as aspirin (COX-1/COX-2 ratio is 0.24) and indomethacin (COX-1/COX-2 ratio is 0.03). Since 2-aminophenol and 2-aminophenoxazine-3-one is expected to have lower adverse side-effects such as stomachache than nonsteroidal COX inhibitors when applied to patients internally, they are useful as internal medicines such as anti-inflammatory, antipyretics and analgesics.

Experiment 4

Inhibiting activity on deglanuration reaction of basocyte

Since it was proved that 2-aminophenonoxazine-3-one (questiomycin A) inhibits liberation of anti-inflammatory mediator from macrophage, the effect on degranulation reaction of basocyte, which is known to liberate anti-inflammatory mediator, was investigated likewise. Degranulation rate (%) and inhibition rate of degranulation (%) were calculated based on the measurements of β-hexosaminidase activity in the granules released from the cells by degranulation reaction.

Cells used for measurement of degranulation reaction

RatcancercellsfrombasocyteRBL-2H3 (catalog number "JCRB0023", commercialized by Health Science Research Resources Bank of Japan Health Sciences Foundation), known to be degranulated by cross-linkage of IgE, were used after cultured in a MEM medium (commercialized by Nissui Pharmaceutical Co., Ltd.) supplemented with 10% (v/v) FCS.

Induction of degranulation reaction and measurement of β-hexosaminidase activity

The above cells were recovered from a flask by conventional trypsin-EDTA treatment, then suspended in a MEM medium supplemented with 10% (v/v) FCS to give a cell concentration of $5 \times 10^5$ cells/ml. The suspension was inoculated to a 24-well microplate in a volume of 400 $\mu$l per well, cultured in a 5% (v/v) carbon dioxide incubator for 3 to 5 hours, then admixed with 0.625 $\mu$g/ml of anti dinitrophenol (hereinafter abbreviated as "DNP") mouse IgE (commercialized by Sigma-Aldrich, Inc., diluted by MEM medium supplied with 10% (v/v) FCS) in a volume of 100 $\mu$l per well to sensitize the cells by IgE (a final concentration of the anti DNP-IgE was 0.125 $\mu$g/ml). After the cells were cultured in the 5% (v/v) carbon dioxide incubator overnight, the supernatant was removed by suction, and then each well was washed twice with Siraganian buffer (119 mM NaCl, 5 mM KCl, 0.4 mM $MgCl_2$, 25 mM piperazine-N,N''-bis(2-ethane sulfonic acid)(PIPES), 40 mM NaOH, pH 7.2) by 500 $\mu$l per well. After admixed with Siraganian buffer containing 0.1% bovine serum albumin (BSA), 5.6 mM glucose and 1 mM $CaCl_2$ (hereinafter described as "BSA-containing Siraganian buffer) in a volume of 160 $\mu$l per well, the microplate was warmed at 37°C for 15 minutes. As test samples, 2-aminophenoxazine-3-one (questiomycin A), arbutin having skin-whitening effect and oxatomide used as therapeutic agents for allergic diseases (positive control) were diluted with BSA-containing Siraganian buffer to give the concentrations shown in Table 3, any of the test samples was added by 20 $\mu$l per well, then the microplate was warmed at 37°C for 15 minutes (wells with the test samples). The well with any of the test samples was admixed with DNP-albumin (commercialized by Sigma-Aldrich, Inc.) diluted with BSA-containing Siraganian buffer to give a concentration of 50 $\mu$g/ml by 20 $\mu$l per well, and warmed at 37°C for 15 minutes. After the 24-well microplate was cooled on ice for 10 minutes, the supernatant was collected and 100 $\mu$l thereof was injected into a 96-well microplate to measure the β-hexosaminidase activity in the supernatant. As negative control, BL-2H3 cells were cultured and treated in the same way as the wells admixed with the test samples except for that FCS-supplemented MEM medium containing no antibody was used, and the β-hexosaminidase activity in the supernatant was measured (the well without IgE sensitization). After the supernatants were

removed from some of the wells without IgE sensitization, the wells were washed twice with BSA-containing Siraganian buffer and admixed with BSA-containing Siraganian buffer by 200 μl per well. The cells were broken by twice repeating freezing at -80°C and thawing, whole medium containing the broken cells was collected, centrifuged at 1,500 rpm for 10 minutes, and then 100 μl of the supernatant was injected into a 96-well microplate to measure the β-hexosaminidase activity of the whole granule extract.

Assay for β-hexosaminidase activity

Each well of the 96-well microplate injected with any of the supernatant from the wells with the test samples, the supernatant from the wells without IgE sensitization or the whole granule extract described above was admixed with 50 μl of citrate buffer (pH 4.5) containing 1 mM 4-nitrophenyl-N-acetyl-β-glucosaminide (PNAG) as substrate, kept at 37°C for 1 hour, and then the reaction was stopped by adding 50 μl of 0.1 M sodium carbonate (pH 10.5). Absorbance at 405 to 650 nm of the reaction mixture of the wells with the test samples, the wells without IgE sensitization or the whole granule extract and the degranulation rate (%) and the inhibition rate of degranulation (%) was calculated according to the Formula 1 and 2 described below. The inhibition intensity of degranulation is in Table 3 evaluated on 4-phase scale; "High" (inhibition rate was 50% or higher), "Low" (inhibition rate was 30% or higher but less than 50%), "Ineffective" (inhibition rate was -30% or higher but less than 30%) and "Enhancing" (inhibition rate was less than -30%). In Table 3, "QA" means 2-aminophenoxazine-3-one (questiomycin A); "AB", arbutin; and "OX", oxatomide.

Formula 1:

Degranulation rate (%) = [{(the absorbance of well with test sample) - (the absorbance of well without IgE sensitization)}/{(the absorbance of well of whole degranulation extract) - (the absorbance of well without IgE sensitization)}] × 100

Inhibiting rate of degranulation (%) = [{(the degranulation rate without test sample) - (the degranulation rate with test sample)}/(the degranulation rate without test sample)] × 100

Since the degranulation rate was determined by measuring β-hexosaminidase activity as a marker, it was made sure that 2-aminophenoxazine-3-one (questiomycin A), by which β-hexosaminidase activity was strongly lowered, has no effect of direct inhibition on β-hexosaminidase activity by the method described below. The highest concentration of 2-aminophenoxazine-3-one (questiomycin A) used in the above experiment, as the test sample, was diluted by five-fold with BSA-containing Siraganian buffer and the dilute solution was injected into a 96-well microplate by 50 μl per well. Separately, the whole granule extract described above was injected into a 96-well microplate by 50 μl per well. Each well was admixed with 50 μl of 0.1 M citrate buffer (pH 4.5) containing 1mM PNAG and warmed at 37°C for one hour. The reaction was stopped by adding 50 μl of 0.1 M sodium carbonate (pH 10.5), measuring the absorbance at 405 to 650 nm. As control, the same measurement as described above was carried out using BSA-containing Siraganian buffer alone. The inhibition rate of β-hexosaminidase activity was calculated according to the following Formula 3. The resultant data was obtained by averaging the measurement values from 3 wells each of the test sample or the whole granule extract.

Formula 3:

Inhibition rate of β-hexosaminidase activity (%) = [{(the absorbance with control) - (the absorbance with test sample)} / (the absorbance with control)] × 100

Table 3

| Concentration (μM) | Inhibition intensity of degranulation | | |
|---|---|---|---|
| | QA | AB | OX |
| 0.63 | Ineffective | - | - |
| 1.5 | - | - | Ineffective |
| 5 | Low | - | Ineffective |
| 10 | High | - | - |
| 23 | High | - | Low |
| 31.3 | High | Ineffective | - |
| 40 | High | - | - |
| 94 | - | - | High |
| 200 | - | Ineffective | - |
| 500 | - | Ineffective | - |
| 2000 | - | Ineffective | - |
| -: Not tested | | | |

As is evident from Table 3, 2-aminophenoxazine-3-one exerted inhibiting activity on degranulation at a concentration of 5 μM or higher, and the activity was higher at concentrations of 10 to 40 μM. Oxatomide, as positive control, exerted inhibiting activity on degranulation at a concentration of 23 μM or higher, and the activity is higher at the concentration of 94 μM. Arbutin exert no inhibition activity on degranulation. When 40 μM 2-aminophenoxazine-3-one was added, inhibition rate of β-hexosaminidase activity was -9.9%. When DMSO, as the solvent, was added at the concentration in the medium, the inhibition rate of β-hexosaminidase activity was -8.6%. Since it was proved that 2-aminophenoxazine-3-one alone and DMSO do not inhibit β-hexosaminidase activity, it was revealed that 2-aminophenoxazine-3-one inhibits the degranulation reaction of basocyte itself. These results indicated that 2-aminophenol and the derivatives of 2-aminophenol such as 2-aminophenoxazine-3-one are useful as antiallergic agent because they inhibit degranulation reaction of basocyte or mast cell. Since it is presumable that it also inhibits releasing mediators involved in inflammation following degranulation reaction, 2-aminophenol and 2-aminophenoxazine-3-one may inhibit inflammatory reaction mediated by basocyte or mast cell as well as inflammatory reaction mediated by macrophage. Although no concrete data was shown, DMSO, used as solvent for the test samples, showed no cytopathy affecting the measurement results in the culture conditions of the this experiment.

Experiment 5

Inhibiting effect of 2-aminophenol and 2-aminophenoxazine-3-one on melanin synthesis

Mouse melanoma cell line B16 (ATCC:CRL-6322) was suspended in RPMI1640 medium supplemented with 10% (v/v) FCS and inoculated into a 6-well plate by 2.0 × $10^4$ cells per well. After the cells adhered to the wells, 2-aminophenoxazine-3-one was added to the wells to give a concentration of 0.5, 1 or 2 μM, and 2-aminophenol was added to give a concentration of 1, 2 or 4 μM, or kojic acid as positive control was added to give a concentration of 0.5, 1 or 2 mM, then the cells were cultured in 5% $CO_2$ atmosphere at 37°C for 5 days in conventional manner. As control, a system with no test sample was provided. The number of living cells in each sample collected after cultivation was

counted, the cells were washed with phosphate buffer, dissolved in 1N NaOH solution, boiled for 30 minutes, and measured for its absorbance at 450 nm with a commercial plate reader. The amount of melanin in each sample was calculated with a calibration curve previously drawn by plotting the absorbance of standard melanin (commercialized by SIGMA, Inc.) at the same wavelength. The amount of protein was measured by conventional Bradford method and the amount of melanin per 1 mg of protein was calculated. The amount of melanin (%) was calculated according to Formula 4, and the living cell rate (%) was calculated according to Formula 5. The results are in Table 4.

Formula 4:

Amount of melanin (%) = {(the amount of melanin per 1mg of protein with test sample) / (the amount of melanin per 1mg of protein in control)} × 100 (%)

Formula 5:

Living cell rate (%) = {(the number of living cell with test sample) / (the number of living cell in control)} × 100 (%)

Table 4

| Test sample | | Amount of Melanin (%) | Living cell rate (%) |
|---|---|---|---|
| Koji acid | 0.5 mM | 79 | 93 |
| | 1 mM | 71 | 86 |
| | 2 mM | 59 | 73 |
| 2-Aminophenoxazin-3-one | 0.5 μM | 74 | 107 |
| | 1 μM | 69 | 95 |
| | 2 μM | 61 | 77 |
| 2-Aminophenol | 1 μM | 62 | 95 |
| | 2 μM | 55 | 79 |
| | 4 μM | 50 | 52 |

As shown in Table 4, every sample inhibited the growth of B16 cell. In the concentration at which the cell-growth was not inhibited, the inhibiting effect on melanin production of 2-aminophenozazin-3-one or 2-aminophenol was 1,000-fold higher than that of kojic acid.

Experiment 6

Effect of combinational use with other agents

By the same way as described in Experiment 5, effect of concomitant use of 2-aminophenoxazine-3-one with kojic acid, trehalose or ascorbic acid glucoside was investigated. Mouse melanoma cell line B16 (ATCC:CRL-6322) was suspended in RPMI1640 medium supplemented with 10% (v/v) FCS and inoculated to 6-well plate by $2.0 \times 10^4$ cells per well. After the cells adhered to the wells, 2-aminophenoxazine-3-one, kojic acid, trehalose ("TREHA" commercialized by Hayashibara Shoji, Inc., Okayama, Japan) or ascorbic acid 2-glucoside ("AA2G" commercialized by Hayashibara Biochemical Laboratories, Inc., Okayama, Japan) was added to the well to give the concentrations described in Table 5, then cultured by conventional method in 5% $CO_2$ atmosphere at 37°C for 5 days. As control, a system with no test sample was provided. The cells after cultivation were collected, washed with phosphate buffer, dissolved in 1N NaOH

solution, boiled for 30 minutes, and measured for its absorbance at 450 nm with a commercial plate reader. The amount of melanin in the sample was calculated with the calibration curve previously drawn by plotting the absorbance of standard melanin (commercialized by SIGMA, Inc.) at the same wavelength. The amount of protein was measured by conventional Bradford method and the amount of melanin per 1 mg of protein was calculated. The amount of melanin (%) was calculated according to Formula 4. The results are in Table 5.

Table 5

| | 2-Aminophenoxsazine -3-one (μM) | Kojiacid (mM) | Trehalose (mM) | Ascorbic acid 2-glucoside (mM) | Amount of melanin (%) |
|---|---|---|---|---|---|
| 1 | 0.5 | - | - | - | 74 |
| 2 | 1 | - | - | - | 69 |
| 3 | 2 | - | - | - | 61 |
| 4 | - | 0.5 | - | - | 79 |
| 5 | - | 1 | - | - | 71 |
| 6 | - | 2 | - | - | 59 |
| 7 | - | - | 20 | - | 82 |
| 8 | - | - | 40 | - | 75 |
| 9 | - | - | 80 | - | 66 |
| 10 | - | - | - | 2 | 80 |
| 11 | - | - | - | 4 | 68 |
| 12 | - | - | - | 8 | 63 |
| 13 | 0.25 | 0.25 | - | - | 73 |
| 14 | 0.5 | 0.5 | - | - | 69 |
| 15 | 1 | 1 | - | - | 56 |
| 16 | 0.25 | - | 10 | - | 76 |
| 17 | 0.5 | - | 20 | - | 62 |
| 18 | 1 | - | 40 | - | 54 |
| 19 | 0.25 | - | - | 1 | 74 |
| 20 | 0.5 | - | - | 2 | 61 |
| 21 | 1 | - | - | 4 | 54 |

As shown in Table 5, kojic acid, trehalose, and ascorbic acid 2-glucoside, which exert inhibiting effect on melanin production even when used alone, synergistically exert inhibiting effect on melanin production when used with 2-aminophenoxsazine-3-one. When they are combinationally used, their cytotoxicities were reduced and exerted inhibiting effect on melanin production with maintaining a high living cell level.

Experiment 7

Panel test (external dermatological agent)

A panel test was performed to investigate whether the external dermatological agent of the present invention exerts anti-inflammatory effect and inhibiting effect on melanin synthesis in sunburnt skin. Twenty panelists were put with a light-blocking adhesive tape having an area of about 10 $cm^2$ on their arms to avoid sunlight exposure and allowed to bath in the sea half a day. After bathing, the tapes were taken off and the condition of the area of the skin in each panelist was regarded as the condition before sunburn. An adequate amount of a milky lotion containing 2-aminophenoxazine-3-one (Sample A) or 2-aminophenol (Sample B) of Example 1 described later or the milky lotion without 2-aminophenoxazine-3-one and 2-aminophenol as control was applied to the sunlight-exposed skin of the arm in each panelist twice a day. The condition of the inflammation of the skin (pain and swelling) after 3 days, and the condition of melanin deposit (brownness of the skin) after 7 days were observed glossy by a doctor. The condition was evaluated based on 4-phase scale; "Effective" (restored as before sunburn), "Mildly effective" (effective than control lotion although restored not as before sunburn), "Ineffective" (same as control lotion), and "Adversely effective" (aggravated than control lotion) . The results are in Table 6.

Table 6

| Evaluation | Inhibition of melanin synthesis | | Anti-inflammatory effect | | | |
|---|---|---|---|---|---|---|
| | | | Pain alleviation | | Swelling alleviation | |
| | Sample A | Sample B | Sample A | Sample B | Sample A | Sample B |
| Effective | 5 | 3 | 11 | 8 | 9 | 7 |
| Mildly effective | 9 | 10 | 6 | 7 | 9 | 9 |
| Ineffective | 6 | 7 | 3 | 5 | 2 | 4 |
| Adversely effective | 0 | 0 | 0 | 0 | 0 | 0 |

As shown in Table 6, 70% of the panelists were evaluated their inhibition of melanin production of Sample A (2-aminophenoxazine-3-one) as "Effective" or "Mildly effective", 85 to 90% of the panelists were evaluated the anti-inflammatory effect thereof as "Effective" or "Mildly effective". Sixty five percent of the panelists were evaluated the inhibition of melanin production of Sample B (2-aminophenol) as "Effective" or "Mildly effective", 75 to 80% of the panelists were evaluated the anti-inflammatory effect thereof as "Effective" or "Mildly effective". These results indicate that the agents comprising 2-aminophenoxazine-3-one or 2-aminophenol have excellent inhibiting effect on melanin synthesis and anti-inflammatory effect.

Experiment 8

Therapeutic effects on gingivitis

A panel test was performed to investigate whether the anti-inflammatory agent of the present invention exerts therapeutic effects on gingivitis. Eighteen patients with gingivitis, not improved by tooth-brushing alone after each meal, were divided into three groups. Two groups were allowed to rinse their mouths with the agent comprising 2-aminophenoxazine-3-one (Sample A) or 2-aminophenol (Sample B) in later described Example 4 after tooth-brushing after each meal, and the other group rinsed their mouths with the agent without 2-aminophenoxazine-3-one and 2-aminophenol. After 2 weeks, the condition of gingivitis was observed glossy by a doctor. The patients' conditions were evaluated based on 4-phase scale; "Effective" (recovered from gingivitis), "Mildly effective" (alleviated of gingivitis compared to before the test), "Ineffective" (same as before the test) and "Adversely effective" (aggravated compared to before the test). The results are shown in Table 7.

Table 7

| Evaluation | Sample A | Sample B | Control |
|---|---|---|---|
| Effective | 1 | 0 | 0 |
| Mildly effective | 5 | 5 | 2 |
| Ineffective | 0 | 1 | 3 |
| Adversely effective | 0 | 0 | 1 |

As is evident from Table 7, gingivitis was alleviated in the patients when Sample A or Sample B was used. These results indicate that 2-aminophenoxazine-3-one and 2-aminophenol are effective as therapeutic agents for gingivitis.

Experiment 9

Therapeutic effects on gastritis

A panel test was performed to investigate whether the anti-inflammatory agent of the present invention exerts therapeutic effects on gastritis. Twelve patients of gastritis were divided into three groups. Two groups were administered with the tablet comprising 2-aminophenoxazine-3-one (Sample A) or 2-aminophenol (Sample B) in later described Example 5 after eachdinner, and the other group was administered with a tablet without 2-aminophenoxazine-3-one and 2-aminophenol. After a week, the condition of gastritis was diagnosed with interview by a doctor. The patients' conditions were evaluated based on 4-phase scale; "Effective" (recovered from gastritis), "Mildly effective" (alleviated of gastritis compared to before the test), "Ineffective" (same as before the test) and "Adversely effective" (aggravated compared to before the test). The results are shown in Table 8.

Table 8

| Evaluation | Sample A | Sample B | Control |
|---|---|---|---|
| Effective | 1 | 0 | 0 |
| Mildly effective | 3 | 2 | 0 |
| Ineffective | 0 | 2 | 2 |
| Adversely effective | 0 | 0 | 2 |

As is evident from Table 8, Sample A and B exerted therapeutic effects on gastritis. The results in Experiment 7 and 8 indicate that 2-aminophenoxazine-3-one and 2-aminophenol are effective as therapeutic agents for inflammatory diseases including gingivitis and gastritis.

Experiment 10

Enhancing effect on collagen production

The enhancing effect of 2-aminophenoxazine-3-one on collagen production in the presence of ascorbic acid was investigated by using NHDF, normal fibroblast from human fetus. The effect of 2-aminophenoxazine-3-one alone was investigated. Also, whether 2-aminophenoxazine-3-one causes cytopathy was investigated.

Preparation of the samples

Four-mill molar 2-aminophenoxazine-3-one (dissolved in DMSO) was diluted with Dulbecco's MEM medium (commercialized by Nissui Pharmaceutical Co., Ltd., hereinafter abbreviated as "D-MEM) supplemented with 10 (v/v) % FCS to obtain test samples with the concentrations in the cell culture media described in Table 9.

Measurement of collagen production

MDHF cells (commercialized by Kurabo Industries Ltd., Osaka, Japan, catalog No. "KF-4001") were suspended in D-MEM supplemented with 10 (v/v) % FCS to give a concentration of $5 \times 10^5$ cells/ml, and the suspension was sown in 96-well microplate by 50 μl per well. L-Ascorbic acid 2-glucoside ("ASCOFRESH" commercialized by Hayashibara Shoji, Inc., hereinafter abbreviated as "AA-2G"), which is more stable in media than L-ascorbic acid, was used as ascorbic acid. After cultured at 37°C for a day, the supernatant was removed and any one of the samples and D-MEM medium supplemented with 10 (v/v) % FCS containing 100 μM AA-2G (a final concentration of 50 μM) or D-MEM medium supplemented with 10 (v/v)% FCS not containing AA-2G were added by 100 μl per well, and cultured at 37°C for 3 days in 5 (v/v)% CO2 atmosphere. After the culture supernatants were removed, newly prepared test sample with the same concentration as that of the initially added solution and newly prepared D-MEM medium with or without 100 μM AA-2G were added by 100 μl each per well, and cultured at 37°C for 3 days in 5 (v/v)% $CO_2$ atmosphere. As control, the cells were cultured in the same way except that D-MEM medium supplemented with 10 (v/v) % FCS alone or D-MEM medium supplemented with 10 (v/v)% FCS containing AA-2G alone was added. In all the cell cultures, the concentration of DMSO used to dissolving 2-aminophenoxazine-3-one was adjusted to the final concentration of 0.05 (v/v)%. After the culture supernatant was removed, 1 M acetate solution containing 1 mg/ml of pepsin (commercialized by SIGMA, Inc.) was added by 50 μl per well and the plate was shaken at room temperature for 4 hours with a plate mixer for pepsin digestion.

The digest was collected in a 1.5 ml-tube by pipetting, admixed with 200 μl per tube of Dye Reagent of sircol Collagen Assay Kit (commercialized by Biocolor Ltd.), and allowed to react at room temperature for 30 minutes by inversely mixing. The supernatant of the reaction mixture was completely removed by centrifugation (4°C, 15,000rpm, 10 minutes), and after the precipitate was dissolved in 50 μl of 1 N NaOH, the resulting supernatant was measured for its absorbance at 560nm to 650 nm. Separately, collagen (commercialized by KOKEN Co., Ltd.) was added to a 96-well microplate to give an amount of 5 μg to 0.0395 μg per well by 2-step dilution, treated with pepsin, the resulting digest was treated by the same method as the cell removal from the supernatant and measured for its absorbance at 560nm to 650 nm to make a standard curve. According to the standard curve, the amount of collagen in the pepsin digest of the cells was calculated. The results are in Table 9. In the test, tree wells were used for determining the concentration of respective test samples and the data were averaged. "QA" in Table 9 means 2-aminophenoxazine-3-one (questiomycin A).

Cytopathic test

Cells were cultured with test samples and D-MEM medium supplemented with 10 (v/v)% FCS with or without AA-2G for 6 days by the same method as above, and the supernatant was removed, washed with phosphate buffered saline (PBS(-)), and admixed with alamar Blue (commercialized by TREK Diagnostic Systems, Inc.), which had been diluted by 10-fold with D-MEM medium in a volume of 200 μl per well. After culturing at 37°C for 90 minutes, the fluorescence intensity (exciting light: 544 nm, fluorescence: 590 nm) was measured. The cell survival rate (%) was calculated by dividing the fluorescence intensity of a well with a test sample by the fluorescence intensity of a well with a control sample, and multiplying by 100. The results are shown in Table 9.

Table 9

| QA concentration (μM) | Collagen production (μg/well) | | Cell survival rate (%) | |
|---|---|---|---|---|
| | AA-2G addition | AA-2G free | AA-2G addition | AA-2G free |
| 0 | 0.78 ± 0.16 | 0.54 ± 0.08 | 100 | 100 |
| 0.125 | 1.32 ± 0.45 | 0.66 ± 0.05 | 100 | 101 |
| 0.25 | 1.64 ± 0.21** | 0.64 ± 0.06 | 100 | 103 |
| 0.5 | 1.57 ± 0.18* | 0.63 ± 0.07 | 92 | 99 |
| 1.0 | 1.03 ± 0.22 | 0.71 ± 0.10 | 79 | 92 |
| 2.0 | 0.58 ± 0.06 | 0.70 ± 0.16 | 73 | 88 |
| *: P < 0.05 **: P<0.01 | | | | |

As is evident from Table 9, when cultured in a medium with 2-aminophenoxazine-3-one and AA-2G, collagen production increased depending on 2-aminophenozazine-3-one concentration in the range of 0.125 to 0.25 μM. The amount of produced collagen is more or less the same at the QA concentration of 0.25 to 0.5 μM. The amount of produced collagen more reduced at the QA concentration of μM or more than at the QA concentration of 0.25 and 0.5 μM. On the other hand, when AA-2G was not contained, any enhancement of collagenproductionwas not observed. 2-Aminophenoxazine-3-one with the concentration of 0.5 μM or lower, at which the cell survival rate was 92% or higher, which is different from control by only 8% or lower, was not considered to be cytopathic. However, when the concentration was 1 μM or higher, it gives cytopathy depending on the concentration. Therefore, when the concentration of 2-aminophenoxazine-3-one was 1 μM or higher, collagen production reduced because of the cytopathy on NHDF cell.

These results indicate that 2-aminophenol and the derivatives of 2-aminopehnol such as 2-aminophenoxazine-3-one enhance collagen production at the concentration of 0.125 μM or higher in the presence of ascorbic acid, and the effect is remarkable at the concentration of 0.25 μM. These results indicate that the external dermatological agent comprising 2-aminophenol or its derivatives such as 2-aminophenoxazine-3-one is effective in enhancing collagen production and useful as antiwrinkle or antiaging agent as well as anti-inflammatory or whitening agent.

The following examples explain the preferred embodiments of the present invention.

Example 1

Milky lotion

| | | |
|---|---|---|
| A: | Alkyl polymer of acrylic acid and meta acrylic acid | 0.2 w/w % |
| | Xanthan gum | 0.2 w/w % |
| | Purified water | 73.0 w/w % |
| B: | Glycerol | 3.0 w/w % |
| | Ethanol | 17.0 w/w % |
| | Sodium hydroxide | 0.05 w/w % |
| | Purified water | 2.5 w/w % |
| | Magnesium ascorbate phosphate | 3.0 w/w % |
| | Disodium edetate | 0.05 w/w % |
| | 2-Aminophenoxazine-3-one prepared in Experiment 1 or 2-aminophenol (commercialized by Wako Pure Chemical Industries, Ltd., Osaka, Japan) | 1.0 w/w % |

According to conventional method, the ingredients of A were homogeneously mixed by heating, cooled, and admixed with the ingredients of B to make into an external dermatological agent.

The agent has beneficial anti-inflammatory effect and usefulness for therapy of atopy because it has inhibiting effect on degranulation of basocyte or mast cells. Since the agent inhibits skin inflammation caused by sunburn, dullness, spot, freckle and wrinkles by inhibiting melanin formation and enhancing collagen production, it is a milky lotion for making skin clear and beautiful.

Example 2

Pack

| | | |
|---|---|---|
| A: | Polyvinyl alcohol | 16.0 w/w % |
| | Silicic anhydride | 0.5 w/w % |
| | Polyethyleneglycol | 0.5 w/w % |
| | Polyoxypropylenemethylglucoside | 5.0 w/w % |
| | Glycerol | 5.0 w/w % |
| | Purified water | 60.94 w/w % |
| B: | Ethyl alcohol | 10.10 w/w % |
| | Antiseptic | 0.01 w/w % |
| C: | Disodium edetate | 0.05 w/w % |
| | 2-Aminophenoxazine-3-one prepared in Experiment 1 or 2-aminophenol (commercialized by Wako Pure Chemical Industries, Ltd., Osaka, Japan) | 1.0 w/w % |
| | Purified water | 1.0 w/w % |

Ingredients of A were mixed, dissolved by heating at 70°C, and admixed with the ingredients of B. After cooled, the ingredients of C were dispersed therein to obtain a pack.

The product has beneficial anti-inflammatory effect and usefulness for therapy of atopy because it has inhibiting effect on degranulation of basocyte or mast cells. Since the product inhibits skin inflammationcausedby sunburn, dullness, spot, freckle and wrinkles by inhibiting melanin production and enhancing collagen production, it is a pack for making skin clear and beautiful.

Example 3

Toothpaste

A toothpaste was prepared according to the following formula.

| | |
|---|---|
| Insoluble sodium metaphosphate | 26.0 w/w % |
| Glycerol | 25.0 w/w % |

(continued)

| | |
|---|---|
| Dicalcium phosphate | 15.0 w/w % |
| Sodium lauryl sulfate | 1.5 w/w % |
| Tragacanth gum | 1.4 w/w % |
| Flavor | 1.0 w/w % |
| 2-Aminophenoxazine-3-one prepared in Experiment 1 or 2-aminophenol (commercialized by Wako Pure Chemical Industries, Ltd., Osaka, Japan) | 0.1 w/w % |
| Saccharin | 0.1 w/w % |
| Sodium copper chlorophyllin | 1.0 w/w % |
| Water | 28.9 w/w % |

The product is useful to reduction of gingivitis and therapy or prevention of periodontal disease or pyorrhea.

Example 4

Liquid agent

2-Amnophenoxazine-3-one was dissolved to give a concentration of 1 μg/ml, or 2-aminophenol was dissolved to give a concentration of 0.1 μg/ml in saline containing 1 (w/w)% trehalose as stabilizer, and the solution was finely filtrated and sterilized in a conventional manner to obtain a liquid agent.

The agent, which has inhibiting effect on COX, NO synthesis and degranulation, is useful as a therapeutic, preventive, analgesic and antipyretic agent for various inflammatory diseases such as rheumatism, periodontal disease, osteoporosis, gastritis and atopy, cancer and Alzheimer's disease in the form of an internal agent, injection drug or mouth wash.

Example 5

Tablet

2-Aminophenoxazine-3-one or 2-aminophenol was admixed with anhydrous crystalline α-maltose powder ("FINETOSE", commercialized by Hayashibara Shoji, Inc., Okayama, Japan) and made into a 200-mg tablet containing 1 μg of 2-aminophenoxazine-3-one or 0.1 μg of 2-aminophenol.

The tablet, which has inhibiting effect on COX, NO synthesis and degranulation, is useful as a therapeutic, preventive, analgesic and antipyretic agent for various inflammatory diseases such as rheumatism, periodontal disease, osteoporosis, gastritis and atopy, cancer and Alzheimer's disease when orally administered.

## INDUSTRIAL APPLICABILITY

As described above, 2-aminophenol and derivatives thereof, having inhibiting activities of NO production, PGE2 production and degranulation, are useful as anti-inflammatory or antiallergic agents. Since they also have inhibiting effect on melanin synthesis and enhancing effect on collagen production, they are useful as external dermatological agents for skin-whitening, anti-wrinkle skin care, and antiaging.

## Claims

1. An anti-inflammatory agent comprising 2-aminophenol or a derivative thereof as an effective ingredient.

2. The anti-inflammatory agent of claim 1, which inhibits nitric oxide synthesis, cyclooxygenase activity, and degranulation reaction.

3. The anti-inflammatory agent of claim 1 or 2, which is in the form of an external dermatological agent for skin-whitening and/or an anti-wrinlke skin care.

4. The anti-inflammatory agent of any one of claims 1 to 3, wherein said derivative of 2-aminophenol is 2-aminophenoxazine-3-one.

## INTERNATIONAL SEARCH REPORT

International application No.
PCT/JP2007/070077

A. CLASSIFICATION OF SUBJECT MATTER
*A61K31/136*(2006.01)i, *A61K8/41*(2006.01)i, *A61K8/49*(2006.01)i, *A61K31/5415*(2006.01)i, *A61P29/00*(2006.01)i, *A61P43/00*(2006.01)i, *A61Q19/02*(2006.01)i

According to International Patent Classification (IPC) or to both national classification and IPC

B. FIELDS SEARCHED

Minimum documentation searched (classification system followed by classification symbols)
A61K31/136, A61K8/41, A61K8/49, A61K31/5415, A61P29/00, A61P43/00, A61Q19/02

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)
CAPLUS(STN), REGISTRY(STN), MEDLINE(STN), BIOSIS(STN), EMBASE(STN)

C. DOCUMENTS CONSIDERED TO BE RELEVANT

| Category* | Citation of document, with indication, where appropriate, of the relevant passages | Relevant to claim No. |
|---|---|---|
| X<br>A | JP 2005-60325 A (Akio TOMOTA),<br>10 March, 2005 (10.03.05),<br>Full text<br>(Family: none) | 1,2,4<br>3 |
| X | JP 8-175959 A (Kao Corp.),<br>09 July, 1996 (09.07.96),<br>Full text<br>(Family: none) | 3 |
| A | JP 2005-272334 A (Akio TOMOTA),<br>06 October, 2005 (06.10.05),<br>Full text<br>(Family: none) | 1-4 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 05 November, 2007 (05.11.07) | 13 November, 2007 (13.11.07) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

International application No.
PCT/JP2007/070077

C (Continuation). DOCUMENTS CONSIDERED TO BE RELEVANT

| Category* | Citation of document, with indication, where appropriate, of the relevant passages | Relevant to claim No. |
|---|---|---|
| A | JP 2004-143101 A (Akio TOMOTA), 20 May, 2004 (20.05.04), Full text (Family: none) | 1-4 |
| A | JP 2002-193726 A (L'Oreal), 10 July, 2002 (10.07.02), Full text & EP 1205179 A1 & FR 2816502 A1 & US 2002/107282 A1 | 1-4 |
| A | JP 2000-510490 A (L'Oreal), 15 August, 2000 (15.08.00), Full text & EP 1001741 A1 & WO 99/32077 A1 & FR 2772607 A1 & US 6365135 B1 | 1-4 |
| A | JP 2000-508351 A (L'Oreal), 04 July, 2000 (04.07.00), Full text & WO 99/10318 A1 & EP 966434 A1 & FR 2767823 A1 & US 6423854 B1 & US 2002/161040 A1 | 1-4 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- JP 21431012004 B
- JP 2005272334 A
- JP 2005060325 A
- JP 2003002878 A

### Non-patent literature cited in the description

- **Shinmura et al.** Possible mechanisms of cyclooxygenase (COX)-2 hazard: Is COX-2 in the cardiovascular system a friend or a foe?. *Inflammation and Regeneration,* 2005, vol. 25 (6), 517-524
- **Motohashi et al.** Potential antitumor phenoxazines. *Medicinal Research Reviews,* 1991, vol. 11, 239-294
- **Shimamoto et al.** Antitumor effects of a novel phenoxazine derivative on human leukemia cell lines in vitro and in vivo. *Clinical Cancer Research,* 2001, vol. 7, 704-708